(19) Europäisches Patentamt
European Patent Office
Office européen des brevets

(11) **EP 3 132 741 B1**

(12) **EUROPEAN PATENT SPECIFICATION**

(45) Date of publication and mention
of the grant of the patent:
**18.03.2020 Bulletin 2020/12**

(51) Int Cl.:
*A61B 5/01* *(2006.01)*    *A61B 5/00* *(2006.01)*
*A61B 5/11* *(2006.01)*

(21) Application number: **16184698.5**

(22) Date of filing: **18.08.2016**

(54) **HEALTH CARE APPARATUS AND METHOD OF OPERATING THE SAME**

GESUNDHEITSPFLEGEVORRICHTUNG UND VERFAHREN ZUM BETRIEB DAVON

APPAREIL DE SOINS DE SANTÉ ET PROCÉDÉ D'UTILISATION CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.08.2015 KR 20150118272**

(43) Date of publication of application:
**22.02.2017 Bulletin 2017/08**

(73) Proprietor: **Samsung Electronics Co., Ltd.**
**Suwon-si, Gyeonggi-do 16677 (KR)**

(72) Inventors:
• **PARK, Jinyoung**
  **16678 Gyeonggi-do (KR)**
• **KIM, Hyunhee**
  **16678 Gyeonggi-do (KR)**
• **JANG, Hyeongseok**
  **16678 Gyeonggi-do (KR)**

(74) Representative: **Grünecker Patent- und Rechtsanwälte**
**PartG mbB**
**Leopoldstraße 4**
**80802 München (DE)**

(56) References cited:
**US-A1- 2011 152 636    US-A1- 2014 169 400**
**US-B1- 6 287 262    US-B2- 6 953 435**

**Description**

BACKGROUND

**1. Field**

The present disclosure relates to a health care apparatus that detects a skin temperature of a user and uses a result thereof, and a method of operating the health care apparatus.

**2. Description of the Related Art**

Diseases such as diabetes and high or low blood pressure are affected by food ingested by an individual. An individual needs to consume meals suitable for his/her physical constitution and current health conditions.

In order to determine which meal is suitable for his/her health condition, an individual may search for a suitable meal by personally recording meal contents and amounts. However, data may be omitted while an individual personally records meal contents, and the individual may feel burdened and suffer from stress regarding the recording of the meal contents.

Meanwhile, energy consumption of a human body is largely divided into a basal metabolic rate, physical activity energy expenditure, and diet induced thermogenesis (DIT). Here, DIT results from a fact that more heat is generated when food is ingested than when the stomach is empty. However, even when the same food is consumed, an amount of DIT may vary by person since people have different health conditions and eating habits.

US patent application US 2014/0169400 A1 teaches a system and method for approximating caloric energy intake and/or macronutrients composition using thermogenesis.

SUMMARY

Provided are health care apparatuses for obtaining diet induced heat information of a user by using a skin temperature of the user, and methods of operating the health care apparatuses.

Provided are health care apparatuses for determining eating habits and health conditions of a user by using the obtained diet induced heat information, and methods of operating the health care apparatuses.

Provided are health care apparatuses providing guides for improving eating habits or health by using diet induced heat information, and methods of operating the health care apparatuses.

Additional aspects will be set forth in part in the description which follows and, in part, will be apparent from the description, or may be learned by practice of the presented exemplary embodiments.

According to an aspect of the present invention, there is provided a method of operating a health care apparatus according to claim 1. Preferred embodiments are subject to the dependent claims.

The skin temperature may be obtained according to heat radiated or conducted from skin of the user.

The determining includes determining a reference temperature that is a criterion for determining the dietary temperature waveform from the temperature waveform. The reference temperature may be a skin temperature of the user while having an empty stomach.

The reference temperature may be an average skin temperature for a certain period of time based on a point of time when the user ingests the food.

The certain period of time may be within 30 minutes.

The obtaining of the diet metabolism information may include: obtaining information about diet induced heat of the user by using the dietary temperature waveform and physical information of the user; and obtaining the diet metabolism information of the user by using the information about the diet induced heat and food information about the food consumed by the user.

The obtaining of the information about the diet induced heat includes determining at least one of a reference temperature, a dietary temperature, and a dietary time from the temperature waveform; obtaining a unit caloric value of the user by using at least one of the reference temperature, the dietary temperature, and the dietary time; obtaining the information about the diet induced heat of the user by using at least one of the unit caloric value of the user and the physical information of the user.

The dietary temperature is higher than the reference temperature.

The dietary time may be within 6 hours.

The diet metabolism information includes a ratio of the information about the diet induced heat with respect to the food information.

The method further includes determining a type of the food by using the diet metabolism information.

The method further includes displaying the type of the food.

The type of the food is displayed in text or a graph.

The method may further include guiding a dietary habit of the user by using the diet metabolism information.

The method may further include detecting the skin temperature.

The method may further include detecting movement of the user, wherein the skin temperature may be detected when a degree of the detected movement is lower than or equal to a reference value.

The skin temperature may be a temperature of skin of at least one of a wrist and an ankle of the user.

According to an illustrative example, a method of operating a health care apparatus is provided, the method including: generating a temperature waveform according to time, based on a skin temperature of a user; determining, from the generated temperature waveform, a dietary temperature waveform according to food consumed by the user; and determining a type of the food consumed by the user by using the dietary temperature waveform.

The determining of the type of the food may include comparing the dietary temperature waveform and a standard temperature waveform corresponding to standard food.

The standard temperature waveform may include at least one of a first temperature waveform corresponding to protein-based food, a second temperature waveform corresponding to carbohydrate-based food, and a third temperature waveform corresponding to fat-based food.

According to another illustrative example, a health care apparatus includes: a temperature obtainer configured to obtain a skin temperature of a user; and a controller configured to generate a temperature waveform according to time based on the skin temperature, determine, from the generated temperature waveform, a dietary temperature waveform according to food consumed by the user, and obtain at least one of diet metabolism information of the user and a type of the food consumed by the user by using the dietary temperature waveform.

The temperature obtainer may include a temperature sensor configured to detect the skin temperature of the user.

The temperature obtainer may include a communication unit configured to receive the skin temperature of the user from an external device.

The skin temperature may be obtained according to heat radiated from skin of the user.

The controller may determine a reference temperature that is a criterion for determining the dietary temperature waveform from the temperature waveform.

The reference temperature may be any one of a skin temperature when the user has an empty stomach and an average skin temperature for a certain period of time based on a point of time when the user ingests the food.

The controller may obtain information about diet induced heat of the user by using the dietary temperature waveform and physical information of the user, and obtain the diet metabolism information of the user by using the information about the diet induced heat and food information about the food consumed by the user.

The controller may determine at least one of a reference temperature, a dietary temperature, and a dietary time from the temperature waveform, obtain a unit caloric value of the user by using at least one of the reference temperature, the dietary temperature, and the dietary time, and obtain the information about the diet induced heat of the user by using at least one of the unit caloric value of the user and the physical information of the user.

The dietary temperature is higher than the reference temperature.

The diet metabolism information may include a ratio of the information about the diet induced heat with respect to the food information.

The controller may determine at least one of a health condition of the user and a type of the food by using the diet metabolism information.

The controller may provide an indicator for guiding a dietary habit of the user by using the diet metabolism information.

The controller may generate the temperature waveform when a degree of movement of the user is lower than or equal to a reference value.

The skin temperature may be a temperature of skin of at least one of a wrist and an ankle of the user.

The controller may obtain the type of the food by comparing the dietary temperature waveform and a standard temperature waveform corresponding to standard food.

The standard temperature waveform may include at least one of a first temperature waveform corresponding to protein-based food, a second temperature waveform corresponding to carbohydrate-based food, and a third temperature waveform corresponding to fat-based food.

According to another illustrative example, a method of operating a health care apparatus is provided, the method including: generating a temperature waveform according to time based on a skin temperature of a user; determining, from the generated temperature waveform, a dietary temperature waveform corresponding to heat generated by food consumed by a user; determining, from the dietary temperature waveform, diet metabolism information of the user; and outputting the diet metabolism information.

The determining the dietary temperature waveform may include determining, from the temperature waveform, a reference temperature, and determining the dietary temperature waveform from the temperature waveform and the reference temperature. The reference temperature may be a skin temperature of the user after the user has not consumed food for a predetermined amount of time.

The determining of the diet metabolism information may include: determining information about diet induced heat of the user by using the dietary temperature waveform and physical information of the user; and determining the diet metabolism information of the user by using the information about the diet induced heat and food information about the food consumed by the user.

The determining of the information about the diet induced heat may include: determining, from the temperature waveform, at least one of a reference temperature, a dietary temperature, and a dietary time; determining a unit caloric value of the user by using the at least one of the reference temperature, the dietary temperature, and the dietary time; and determining the information about the diet induced heat of the user by using at least one of the unit caloric value of the user and the physical information of the user.

According to another illustrative example, a method of operating a health care apparatus is provided, the method including: generating a temperature waveform according to time, based on a skin temperature of a user; determining, from the generated temperature waveform, a dietary temperature waveform corresponding to heat generated by food consumed by the user; determining a type of the food consumed by the user by using the dietary temperature waveform; and outputting the type of food consumed by the user.

The determining the type of the food consumed by the user may include comparing the dietary temperature waveform and a reference temperature waveform corresponding to a reference food.

The reference temperature waveform may include at least one of a first temperature waveform corresponding to a protein-based food, a second temperature waveform corresponding to a carbohydrate-based food, and a third temperature waveform corresponding to a fat-based food.

According to an aspect of the present invention, there is provided a health care apparatus according to claim 6. Preferred embodiments are subject to the dependent claims.

The at least one processor may be further configured to determine, from the temperature waveform, a reference temperature, and determine the dietary temperature waveform from the temperature waveform and the reference temperature.

The at least one processor is further configured to determine information about diet induced heat of the user by using the dietary temperature waveform and physical information of the user, and to determine the diet metabolism information of the user by using the information about the diet induced heat and food information about the food consumed by the user.

The at least one processor may be further configured to determine, from the temperature waveform, at least one of a reference temperature, a dietary temperature, and a dietary time, determine a unit caloric value of the user by using the at least one of the reference temperature, the dietary temperature, and the dietary time, and determine the information about the diet induced heat of the user by using at least one of the unit caloric value of the user and the physical information of the user.

BRIEF DESCRIPTION OF THE DRAWINGS

These and/or other aspects will become apparent and more readily appreciated from the following description of the exemplary embodiments, taken in conjunction with the accompanying drawings in which:

FIG. 1 is a block diagram of a health care apparatus, according to an exemplary embodiment;
FIG. 2 is a graph showing results of measuring a skin temperature, according to an exemplary embodiment;
FIG. 3 is a flowchart of a method of obtaining, by a health care apparatus, diet metabolism information, according to an exemplary embodiment;
FIG. 4A is a reference graph showing an example of a dietary temperature waveform of a dietary temperature, according to an exemplary embodiment;
FIG. 4B are reference graphs showing an example in which a dietary temperature waveform is corrected by a baseline, according to an exemplary embodiment;
FIG. 5 is a flowchart of a method of obtaining diet metabolism information by using a dietary temperature waveform, according to an exemplary embodiment;
FIG. 6 is a flowchart of a method of obtaining information about diet induced heat, according to an exemplary embodiment;
FIG. 7 is a table showing general relationships between diet metabolism information and food;
FIGS. 8A, 8B, 8C, 8D, 8E, 8F, 8G, and 8H are reference diagrams for describing a method of obtaining and providing diet metabolism information, according to an exemplary embodiment;
FIGS. 9A, 9B, and 9C are reference diagrams for describing a method of registering standard food, according to an exemplary embodiment;

FIGS. 10A, 10B, and 10C are reference diagrams for describing a method of displaying tracked diet metabolism information, according to an exemplary embodiment;

FIG. 11 is a flowchart of a method of determining a type of food by using a dietary temperature waveform, according to an exemplary embodiment; and

FIG. 12 is a diagram illustrating an example of a slave and a master, according to an exemplary embodiment.

DETAILED DESCRIPTION

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, some terms may be arbitrarily selected by the applicant, and in this case, the meaning of the selected terms will be described in detail in the detailed description of the invention. Thus, the terms used herein have to be defined based on the meaning of the terms together with the description throughout the specification.

Throughout the specification, when a region is connected to another region, the regions may be directly connected to each other, or may be electrically connected to each other through an intervening device. Also, when a part "includes" or "comprises" an element, unless there is a particular description contrary thereto, the part can further include other elements, not excluding the other elements. In the following description, terms such as "unit" and "module" indicate a unit for processing at least one function or operation, wherein the unit and the block may be embodied as hardware or software or embodied by combining hardware and software.

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects. Expressions such as "at least one of" when preceding a list of elements, modify the entire list of elements and do not modify the individual elements of the list.

A health care apparatus according to an exemplary embodiment may be a user portable apparatus, such as a wearable apparatus. The health care apparatus may be any one of a wrist watch type, a bracelet type, a ring type, and a hair band type apparatus, which has a communication function and a data processing function, or a combination of at least two thereof. Here, it is assumed that the health care apparatus according to an exemplary embodiment is a wrist watch type or a wrist band type, but the exemplary embodiments are not limited thereto.

Also, the health care apparatus may be realized by using one housing or a plurality of housings. When the health care apparatus is realized by using a plurality of housings, a plurality of components may be connected wirelessly or via wires. For example, the health care apparatus may be divided into a first apparatus including a sensor detecting biometric information of a user, which is worn on a wrist of a user, and a second apparatus processing the biometric information. The health care apparatus may be realized by some components of an apparatus performing another function, such as a mobile terminal.

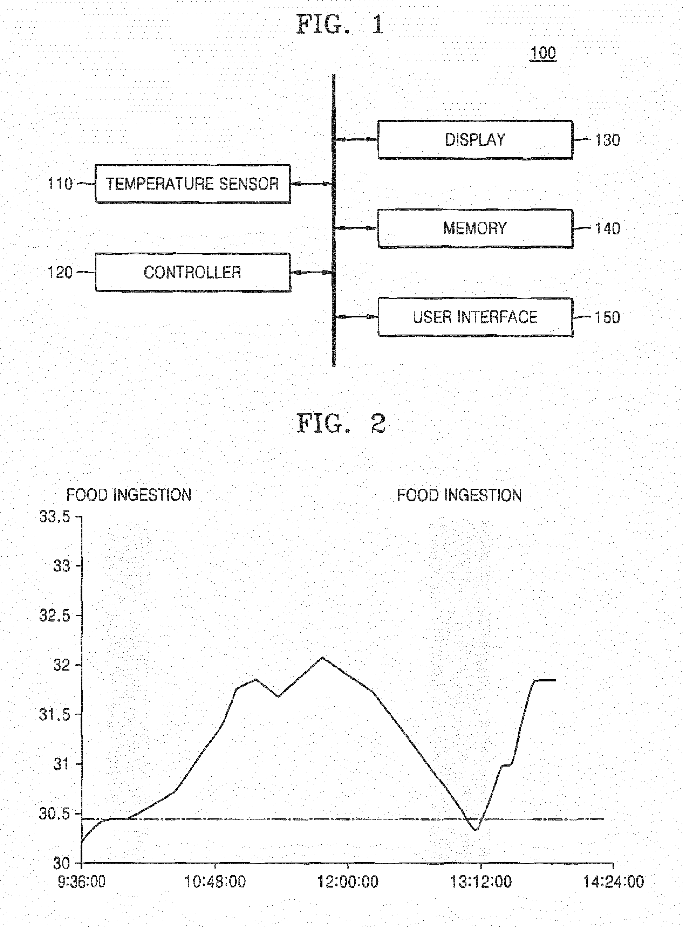

FIG. 1 is a block diagram of a health care apparatus 100, according to an exemplary embodiment. Referring to FIG. 1, the health care apparatus 100 may include a temperature sensor 110 configured to detect a skin temperature of a user, a controller 120 configured to obtain diet metabolism information of the user by using the skin temperature detected by the temperature sensor 110, a display 130 configured to display the diet metabolism information and information related to the diet metabolism information, a memory 140 in which programs usable by the health care apparatus 100 are stored, and a user interface 150 configured to receive a user command. For instance, the controller 102 may obtain the diet metabolism information by determining the diet metabolism information.

The temperature sensor 110 may detect the skin temperature of the user.

Generally, energy of a human body may be largely emitted as work energy and heat energy. About 70% of the energy of the human body is emitted as heat energy. Also, only about 20 to 25% of the energy of the human body is emitted as work energy.

Meanwhile, even if heat is generated, a central temperature of the human body maintains an almost uniform value according to homeostasis, but the skin temperature may change according to activity, meals, body conditions, and environmental factors.

For example, when the user consumes food, heat is emitted as adenosine triphosphate (ATP) is generated in the body according to metabolism of the body. Then, the skin temperature may change according to the emitted heat. In other words, the skin temperature after a meal may be higher than the skin temperature when the user has an empty stomach, e.g., after the user has not consumed food for a predetermined amount of time. Such a higher skin temperature after a meal is due to food, and the skin temperature after a meal may vary according to a type of the food, and eating habits of the user.

Meanwhile, heat of the user may be externally emitted in a form of conduction, convection, radiation, or evaporation. Conduction is affected by material properties of an object contacting a user's skin, and convection and evaporation may be affected by a type and movement of a gas contacting a user's skin. Since radiation uses a thermal exchange method in which heat is directly transferred from skin of a user to an external environment, a thermal balance with the external environment may be maintained when the user is in a resting state (for example, not moving or has no psychological stress). Since radiation energy in a resting state is related to a basal metabolic rate of an individual, a change of basal metabolism may be monitored through the basal metabolism rate in the resting state.

The temperature sensor 110 according to an exemplary embodiment may detect heat emitted by radiation from skin of the user. In other words, the temperature sensor 110 may measure a skin temperature of heat emitted by radiation from the skin. As another example, the temperature sensor 110 may detect the skin temperature via a contact method or a non-contact method.

For example, the temperature sensor 110 may include any type of temperature sensor using a contact method. In the temperature sensor using a contact method, a temperature at a certain point of a user's skin is transmitted to the temperature sensor via thermal conduction, and examples of such a temperature sensor may include a thermistor, an integrated circuit (IC) temperature sensor, a quartz temperature sensor, a surface wave temperature sensor, an optical fiber sensor, and a liquid temperature sensor. According to a resistance measuring method via contact, a temperature coefficient value of electric resistance varies according to a change of a skin temperature, and thus the change of the skin temperature may be monitored by measuring the temperature coefficient value. However, since an energy loss may be generated due to contact, the temperature coefficient value may be monitored by converting the change of the skin temperature into dietary heat or compensating for a certain amount of the energy loss through a change of a relative value.

Also, the temperature sensor 110 may include any type of temperature sensor using a non-contact method. In a non-contact type infrared (IR) sensor, heat is transferred to the non-contact type IR sensor via radiation, and examples of the non-contact type IR sensor include a thermopile and a pyroelectric temperature sensor.

The temperature sensor 110 may detect the skin temperature from, for example, skin of a wrist, a breast, or an ankle of the user. Even when the temperature sensor 110 is worn on the wrist, the breast, or the ankle, the temperature sensor 110 may detect the skin temperature while not contacting the skin of the user. For example, when the user wears the health care apparatus 100, the temperature sensor 110 may be exposed from the health care apparatus 100 and face the skin of the user.

FIG. 2 is a graph showing results of measuring a skin temperature, according to an exemplary embodiment. As shown in FIG. 2, the skin temperature of the user barely changes before a meal, whereas it is increased and then decreased after a meal. The increase in skin temperature may vary according to the metabolic capability of the user and the type of food consumed by the user. In other words, the change in skin temperature may correspond to heat generated by food consumed by the user.

The controller 120 may generate a temperature waveform of the user by using the detected skin temperature. The temperature waveform may be a function indicating a change of the skin temperature according to time. Also, the controller 120 may identify a dietary temperature waveform from the generated temperature waveform. Here, the dietary temperature waveform may be a temperature waveform resulting from heat generated by ingesting food, from among the temperature waveform. For example, the controller 120 may determine a reference temperature from the temperature waveform, and identify the dietary temperature waveform from the reference temperature and the temperature waveform.

In addition, the controller 120 may correct the dietary temperature waveform by using a baseline. The baseline may be a line on which a factor other than food ingestion, such as an external environment or exercise, that may change the skin temperature, is applied. In other words, the baseline may be used to remove an effect of a factor other than diet induced heat from among factors that affect the temperature waveform. Here, the diet induced heat may be heat generated while the user consumes or digests food.

Alternatively, the controller 120 may perform a series of pre-process operations on the temperature waveform before or after identifying the dietary temperature waveform.

For example, the controller 120 may perform a pre-process by applying a baseline correction method on the temperature waveform. The baseline correction method may vary according to a method of filtering an unnecessary noise section (a section affected by a factor other than the skin temperature) from the temperature waveform. For example, any one of a polyline algorithm, a horizontal algorithm, a peak detection algorithm, a linear least squares regression algorithm, a two point algorithm, and a spline algorithm may be used as the baseline correction method.

As another example, the controller 120 may perform a pre-process of offset correction on the temperature waveform. For example, the controller 120 may correct a 2-dimentional (2D) temperature waveform in an x-y axis by a certain value in an y-axis. Here, the certain value may be a value in the y-axis in a section having a uniform value in the temperature waveform. An offset value may change according to a status of the user.

As another example, the controller 120 may perform a pre-process considering a derivative of the temperature waveform. A differential quotient derivative method or a Savitzky-Golay derivative method may be used to consider a derivative. By considering a derivative, information about a metabolic speed and a metabolic change amount of the user may be considered.

Hereinafter, the dietary temperature waveform may denote at least one of a dietary temperature waveform identified from the temperature waveform, a preprocessed dietary temperature waveform, and a dietary temperature waveform corrected via the baseline.

Also, the controller 120 may obtain information about the diet induced heat by using the dietary temperature waveform. In order to obtain the information about the diet induced heat, the controller 120 may use physical information of the user, such as a height, a weight, an age, and a gender. For example, the controller 120 may obtain the information about the diet induced heat by determining the information about the diet induced heat based on the physical information of the user.

Also, the controller 120 may obtain diet metabolism information by using the information about the diet induced heat. The diet metabolism information may include a ratio of the information about the diet induced heat with respect to food information about food consumed by the user. Also, the health care apparatus 100 according to an exemplary embodiment may determine a type of the food consumed by the user or a current health condition of the user by using the diet metabolism information, and guide the user through eating habit information for improving a health condition. Here, the health condition may be information about a dietary balance, a caloric intake, obesity, and a chronic disease.

The display 130 may display information processed by the health care apparatus 100. For example, the display 130 may display a user interface (UI) or a graphical user interface (GUI) for displaying a personalized skin temperature index. The personalized skin temperature index may be displayed in at least one of a number and a graph. The display 130 may include at least one of a liquid crystal display (LCD), a thin-film transistor-liquid crystal display (TFT-LCD), an organic light-emitting diode (OLED), a flexible display, and a 3D display. Also, the health care apparatus 100 may include at least two displays 130 according to an exemplary embodiment.

The display 130 may be configured as a touch screen by forming a mutual layer structure with a touch pad for receiving a user input. When the display 130 is configured as the touch screen by forming the mutual layer structure with the touch pad, the display 130 may be used as an input device as well as an output device. According to an exemplary embodiment, the health care apparatus 100 may automatically start to measure the skin temperature when the display 130 configured as the touch screen detects a touch input of the user in a certain region.

The memory 140 may store data generated while the health care apparatus 100 is operated. The memory 140 according to an exemplary embodiment may be a general storage medium, and may include a hard disk drive (HDD), a read-only memory (ROM), a random access memory (RAM), a flash memory, and a memory card.

The user interface 150 may receive an input from the user for manipulating the health care apparatus 100, or may output information about the diet induced heat obtained by the health care apparatus 100, dietary information based on the diet metabolism information, and information about a health condition.

The user interface 150 may include a button, a keypad, a switch, a dial, or a touch interface for the user to directly manipulate the health care apparatus 100, The user interface 150 may include a display for displaying an image, and may be realized as a touch screen. According to another exemplary embodiment, the user interface 150 may include an input/output (I/O) port for connecting human interface devices (HIDs). The user interface 150 may include an I/O port for input and output of an image.

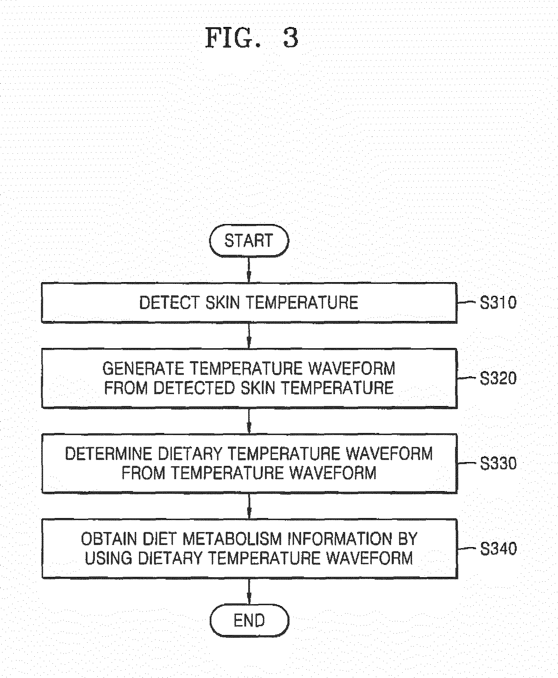

FIG. 3 is a flowchart of a method of obtaining, by the health care apparatus 100, diet metabolism information, according to an exemplary embodiment. Referring to FIG. 3, the temperature sensor 110 may detect a skin temperature in operation S310. The temperature sensor 110 may detect the skin temperature of a user in a non-contact method. The skin temperature may result from heat radiated from skin of the user.

The controller 120 of the health care apparatus 100 may generate a temperature waveform of the user according to time by using the skin temperature received from the temperature sensor 110, in operation S320. The temperature waveform may be a function indicating a change of the skin temperature according to time.

The controller 120 may determine a dietary temperature waveform that is a section in which a temperature is changed by food, from the temperature waveform, in operation S330. In order to determine the dietary temperature waveform, the controller 120 may first determine a reference temperature from the temperature waveform, Here, the reference temperature may be a skin temperature that is not affected by heat generated by food.

For example, the reference temperature may be a skin temperature when the user is in stable state and has an empty stomach or before a meal. In other words, the reference temperature may be a skin temperature after the user has not consumed food for a predetermined amount of time. However, the exemplary embodiments are not limited thereto. A time when heat is generated by food may vary based on a type of the food, a physical constitution of the user, and a measured time, such as during the day or the night.

The reference temperature may be an average skin temperature for a certain period of time based on before and after food ingestion. In other words, the reference temperature may be an average skin temperature of the user during a predetermined period of time based on a point of time when the user consumed the food. For example, the controller 120 may determine an average skin temperature for about 10 minutes before food ingestion as the reference temperature. Alternatively, the controller 120 may determine an average skin temperature for about 5 minutes before food ingestion and for about 5 minutes after the food ingestion as the reference temperature. In addition, the controller 120 may determine an average skin temperature, in which a temperature change is within a certain range based on a point of time when food ingestion starts, as the reference temperature. Alternatively, the controller 120 may determine an average skin temperature, in which a temperature change is within a certain range from the temperature waveform for a certain period of time, for example, for 24 hours, as the reference temperature. Alternatively, when the skin temperature of the user decreases and then increases for a certain period of time after food ingestion, the controller 120 may determine a lowest temperature after the food ingestion as the reference temperature. The skin temperature may temporarily decrease after food ingestion, according to a user. Thus, the controller 120 may variously set the reference temperature based on the user or the measured time.

Moreover, the controller 120 may determine a baseline by referring to an external environment temperature. For example, when a daily temperature difference is high, the skin temperature of the user may change according to diet induced heat after a breakfast, but may also change due to the daily temperature difference. In this case, the reference temperature may be determined based on a point of time before or after a meal, and the baseline may be determined by referring to the external environment temperature. Then, the controller 120 may determine a section in which a temperature is changed by food from the temperature waveform as the dietary temperature waveform. Here, a dietary temperature may be a skin temperature according to heat generated by food consumed by the user. In other words, the dietary temperature may be a skin temperature corresponding to heat generated by food consumed by the user. Generally, the dietary temperature is higher than the reference temperature. Since the skin temperature included in the dietary temperature waveform is higher than the reference temperature, the skin temperature included in the dietary temperature waveform may be the dietary temperature. The dietary temperature may vary according to a type of food consumed by the user. For example, when the food is carbohydrate, the dietary temperature may rapidly increase within a short period of time after food ingestion, and may rapidly decrease within a short period of time. However, when the food is fat, the dietary temperature may slowly increase.

Also, the controller 120 may determine a dietary time. Generally, a time when heat is generated by food may be within about 4 to 7 hours after food ingestion. Thus, the controller 120 may determine about 4 to 7 hours after the food ingestion as the dietary time. Alternatively, the controller 120 may determine a food ingestion section, for example, if food is consumed at 13:00 and then again at 18:00, 13:00 to 18:00 as the dietary time. However, the exemplary embodiments are not limited thereto. The controller 120 may determine any time section of the dietary temperature waveform as the dietary time.

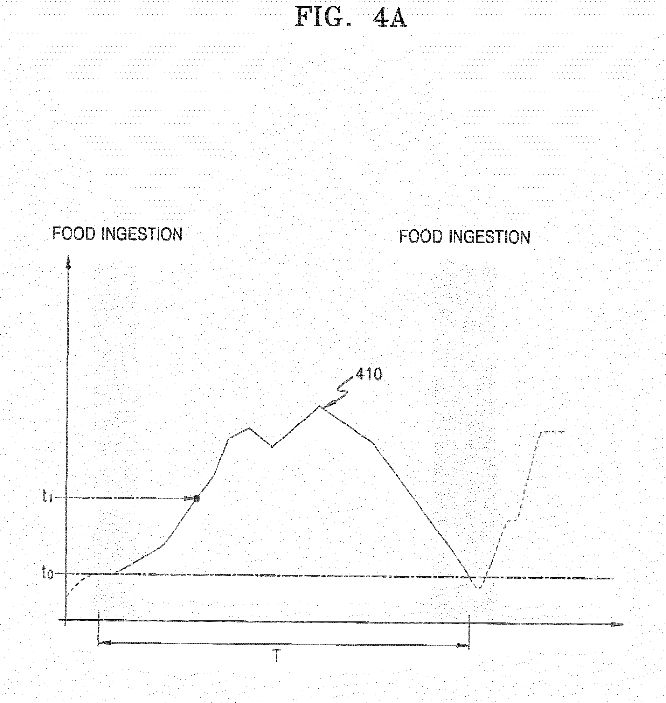

FIG. 4A is a reference graph showing an example of a dietary temperature waveform 410 according to an exemplary embodiment. As shown in FIG. 4A, the controller 120 may determine a reference temperature $t_0$ from a temperature waveform. The reference temperature $t_0$ may be an average skin temperature of a certain section based on a point of time when food is consumed. Also, the controller 120 may determine a waveform having a higher temperature than the reference temperature $t_0$ from among the temperature waveform as the dietary temperature waveform 410. A skin temperature included in the dietary temperature waveform 410 may be a dietary temperature $t_1$. Also, the controller 120 may determine a time section of the dietary temperature waveform 410 as a dietary time T.

Referring back to FIG. 3, the controller 120 may obtain diet metabolism information by using the dietary temperature waveform, in operation S340. When the diet metabolism information is obtained, the controller 120 may use, as well as the dietary temperature waveform, physical information of the user and the food information of the user.

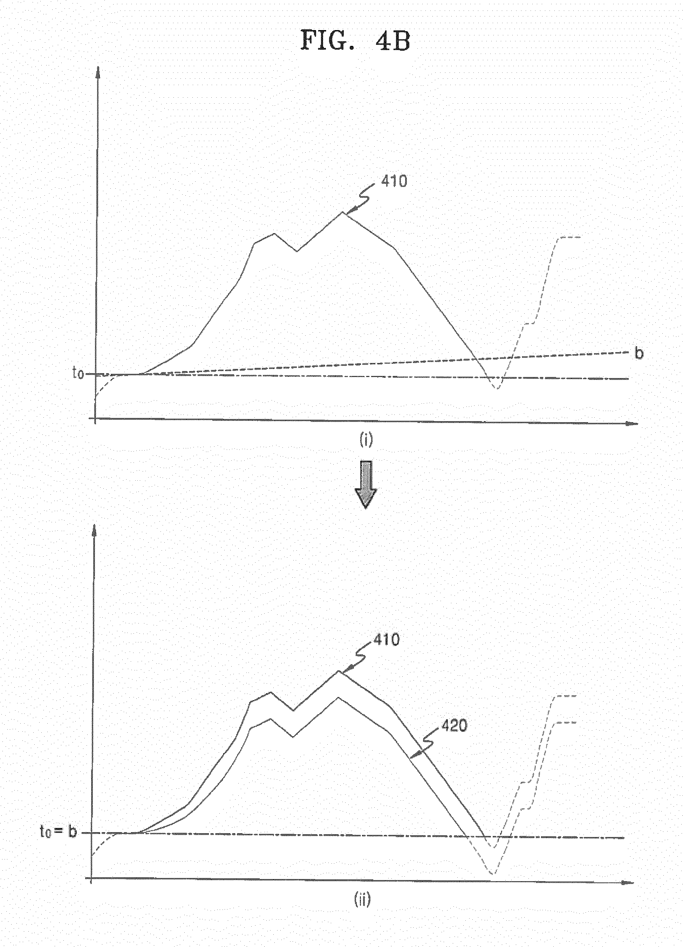

FIG. 4B are reference graphs showing an example in which the dietary temperature waveform 410 is corrected by a baseline b, according to an exemplary embodiment. A skin temperature may change according to an external temperature change or a temperature change due to an exercise, as well as diet induced heat. The controller 120 may determine the baseline b shown in FIG. 4B (i) by using a sensor measuring a temperature of an external environment or a sensor detecting movement of the user. Then, the controller 120 may generate a corrected dietary temperature waveform 420 of FIG. 4B (ii) by correcting the dietary temperature waveform 410 by using the baseline b.

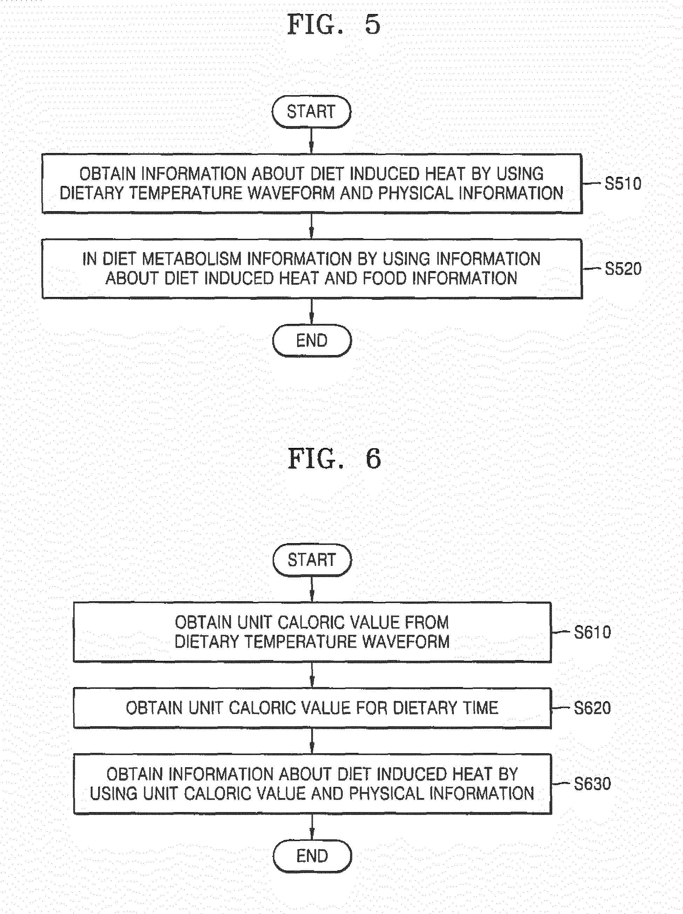

FIG. 5 is a flowchart of a method of obtaining diet metabolism information by using a dietary temperature waveform, according to an exemplary embodiment.

The health care apparatus 100 may obtain information about diet induced heat of a user by using a dietary temperature waveform and physical information of the user, in operation S510. Even when a change of a skin temperature is the same, an amount of the diet induced heat may be different when the physical information, for example, a body surface of the user, is different. Accordingly, the health care apparatus 100 may use the physical information of the user in order to obtain the information about the diet induced heat. A method of obtaining the information about the diet induced heat will be described later.

The health care apparatus 100 may obtain diet metabolism information by using the information about the diet induced heat and food information, in operation S520. The user may input the food information through the user interface 150. The food information may be about a type and an amount, or calories of food consumed by the user. When the food information is input in the type and the amount of the food, the health care apparatus 100 may calculate calories of the food based on the type and the amount of the food. Also, the health care apparatus 100 may obtain the diet metabolism information based on a ratio of the diet induced heat regarding the food information.

FIG. 6 is a flowchart of a method of obtaining information about diet induced heat, according to an exemplary embodiment. As shown in FIG. 6, the health care apparatus 100 may obtain a unit caloric value from a dietary temperature waveform, in operation S610. For example, the controller 120 may determine a reference temperature, a dietary temperature, and a dietary time from the dietary temperature waveform. The reference temperature may be a skin temperature before heat is generated by food. For example, the reference temperature may be a skin temperature of a user when the user has an empty stomach, e.g., after the user has not consumed food for a predetermined amount of time. However, the exemplary embodiments are not limited thereto. The reference temperature may be an average skin temperature for a certain period of time based on before and after food ingestion.

The dietary temperature may be a skin temperature while heat is generated by the food. Generally, the dietary temperature is higher than the reference temperature. The controller 120 may determine a temperature in the dietary temperature waveform as the dietary temperature. The dietary time may be a time when heat is generated by the food, and the controller 120 may determine a time section of the dietary temperature waveform as the dietary time.

The controller 120 may obtain the unit caloric value of the user by using the reference temperature, the dietary temperature, and the dietary time. Here, the unit caloric value may be an amount of heat generated by food per unit surface area of the user.

For example, the controller 120 may obtain the unit caloric value of the user in a unit time by using the reference temperature and an average dietary temperature that is an average of dietary temperatures. For example, the controller 120 may obtain the unit caloric value of the user according to the Stefan-Boltzmann's law. The controller 120 may obtain a unit caloric value $q_r$ according to Equation 1 below.

<Equation 1>

$$q_r = \varepsilon * \sigma * (T^4_s - T^4_a) \quad (W/m^2)$$

Here, $\sigma$ denotes a Stefan-Boltzmann constant, Ts denotes a dietary temperature, Ta denotes a reference temperature, and $\varepsilon$ denotes absorptivity or emissivity of the skin. Absorptivity $\varepsilon$ may vary according to a skin color.

Also, the controller 120 may obtain a unit caloric value $Q_T$ for a dietary time, in operation S620, by multiplying the dietary time T and the unit caloric value $q_r$ according to Equation 2 below.

<Equation 2>

$$Q_T = q_r \times T$$

While calculating a unit caloric value for a dietary time, the health care apparatus 100 may split the dietary time into a plurality of sub-times, obtain a unit caloric value per sub-time, and then calculate the unit caloric value for the dietary time by adding the unit caloric values of the sub-times according to Equation 3 below.

<Equation 3>

$$Q_T = \sum_1^n q_{ri}$$

Here, n denotes the number of sub-times and qri denotes a unit caloric value of an i-th sub-time. By obtaining the unit caloric values by splitting the dietary time, the unit caloric value for the dietary time may be accurately obtained.

The controller 120 may obtain information about diet induced heat of the user by using the unit caloric value for the dietary time and physical information of the user, in operation S630. The controller 120 may obtain a body surface area of the user by using the physical information of the user. For example, the controller 120 may obtain the body surface area of the user by using a weight and a height of the user, according to Equation 4 below.

<Equation 4>

$$S = 0.007184 \times W^{0.425} \times H^{0.725}$$

Here, W denotes a weight of the user in kg and H denotes a height of the user in cm. The body surface area S is given in $m^2$.

Also, the controller 120 may obtain information H about diet induced heat, which is information about heat generated by food during a dietary time, by multiplying a body surface area of the user and a unit caloric value for a dietary time, according to Equation 5 below.

<Equation 5>

$$H = Qr \times S$$

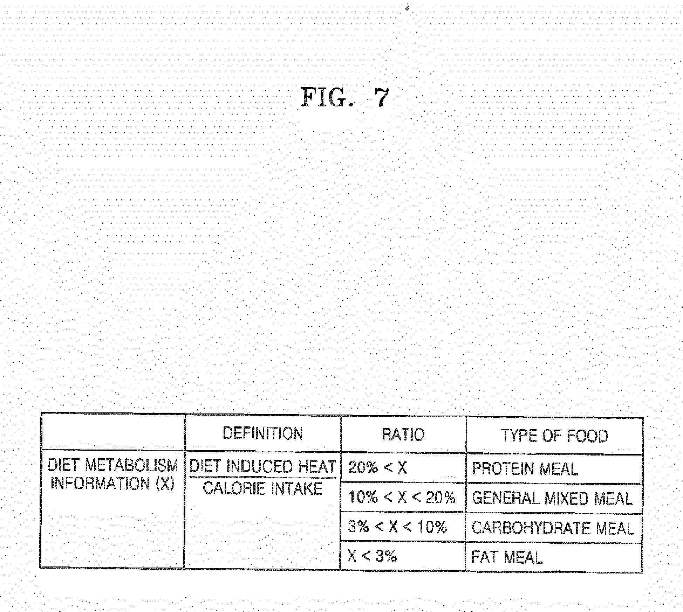

The diet metabolism information described above is related to food. FIG. 7 is a table showing general relationships between diet metabolism information and foods. As shown in FIG. 7, the diet metabolism information may be defined as a ratio of diet induced heat with respect to a calorie intake. When food contains protein, the diet metabolism information is from about 20 to about 30%, when food contains carbohydrate, the diet metabolism information is from about 3 to about 10%, and when food contains fat, the diet metabolism information is from about 0 to about 3%. Accordingly, the health care apparatus 100 may determine a type of food consumed by a user based on the diet metabolism information. Also, when the user does not input food information, since a calorie intake and a caloric value are generally proportional to each other, the type of the food may be determined by using information about a change of a calorie intake according to time.

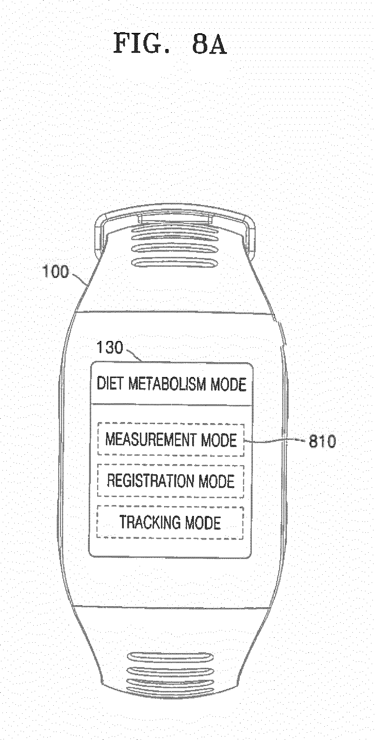

FIGS. 8A through 8H are reference diagrams for describing a method of obtaining and providing diet metabolism information, according to an exemplary embodiment. According to a user input, a mode of the health care apparatus 100 may be set to a measurement mode 810 for measuring diet metabolism. For example, as shown in FIG. 8A, the health care apparatus 100 may provide various diet metabolism modes. A user may input a command of selecting the measurement mode 810 for measuring diet metabolism. Then, the controller 120 of the health care apparatus 100 activates the temperature sensor 110, and the temperature sensor 110 may detect a skin temperature of the user and transmit the skin temperature to the controller 120. Also, the controller 120 may generate a temperature waveform by using the skin temperature. The temperature sensor 110 may detect the skin temperature in real-time. Alternatively, the temperature sensor 110 may detect the skin temperature at regular intervals, for example, every 5 minutes, 10 minutes, 30 minutes, or 1 hour.

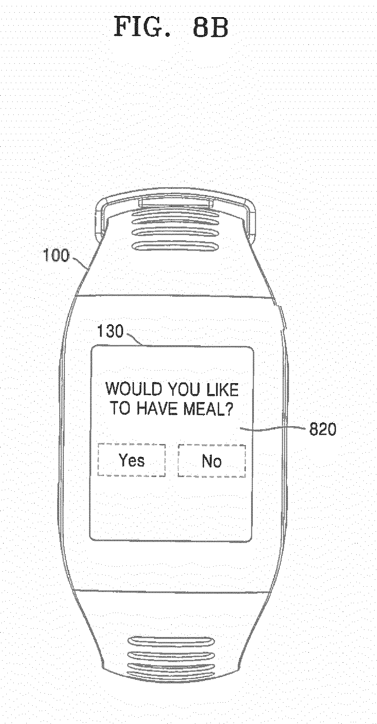

Meanwhile, as shown in FIG. 8B, the health care apparatus 100 may provide a screen 820 for determining whether a meal is started. The skin temperature may change according to heat generated by a meal, but may also change according to another factor, such as a rapid temperature change of an external environment, a psychological change of the user, or a disease. Accordingly, in order to obtain accurate diet metabolism information, the health care apparatus 100 may provide the screen 820 for determining whether a meal is started.

Upon receiving a user input that a meal is started, the health care apparatus 100 may determine a reference temperature based on a point of time when a meal is started, and determine that a change of the skin temperature after starting the meal is due to heat according to the meal. However, the exemplary embodiments are not limited thereto. The health care apparatus 100 may pre-store a temperature database related to a dietary temperature waveform. The controller 120 may determine whether the generated temperature waveform is similar to the dietary temperature waveform stored in the temperature database, thereby determining whether the generated temperature waveform is related the dietary temperature waveform or another factor, such as an external environment or a disease. In this case, the health care apparatus 100 may not provide the screen 820 for determining whether a meal is started.

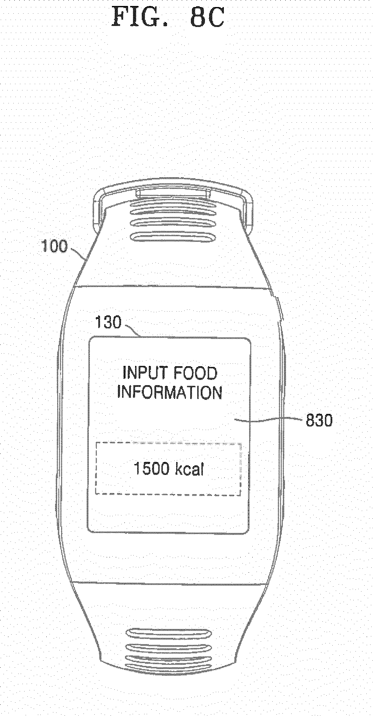

As shown in FIG. 8C, the health care apparatus 100 may provide a screen 830 for receiving food information. The user may input a calorie amount as the food information. However, the exemplary embodiments are not limited thereto, and the user may input a type or amount of food. The health care apparatus 100 may obtain a calorie amount from the type and amount of the food by using a food database in which calories of foods according to types and amounts are stored.

Alternatively, the user may input the food information to the health care apparatus by capturing an image of food to be consumed, Then, the health care apparatus 100 identifies the food from the image, and obtain a calorie of the food by using the food database.

The temperature sensor 110 may detect the skin temperature, and the controller 120 may generate the temperature waveform of the skin temperature and determine a dietary temperature waveform from the temperature waveform. The health care apparatus 100 may generate the temperature waveform for a certain period of time. For example, the certain period of time may be from about 3 hours to about 6 hours. However, the exemplary embodiments are not limited thereto. Even when about 5 hours are required to obtain the diet metabolism information, the health care apparatus 100 may generate the temperature waveform by detecting the skin temperature for about 2 hours. Also, a partial dietary temperature waveform may be determined from the generated temperature waveform, and by comparing the partial dietary temperature waveform and a dietary temperature waveform stored in the temperature database, the remaining dietary temperature waveform of about 3 hours may be predicted and generated.

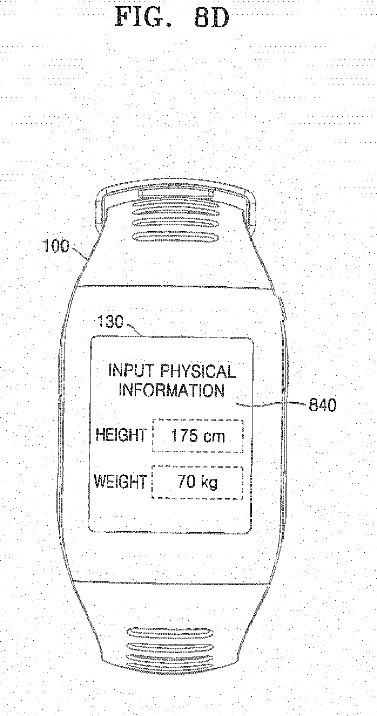

Also, the health care apparatus 100 may receive physical information of the user. For example, as shown in FIG. 8D, the health care apparatus 100 may provide a screen 840 for inputting physical information. The user may input the physical information, such as a height, a weight, an age, and a gender, through the user interface 150.

The health care apparatus 100 may obtain a unit caloric value of the user by using the dietary temperature waveform, obtain information about diet induced heat of the user by using the unit caloric value and the physical information of the user, and obtain diet metabolism information by using the information about the diet induced heat and the food information. The controller 120 may obtain a ratio of the information about the diet induced heat with respect to the food information as the diet metabolism information.

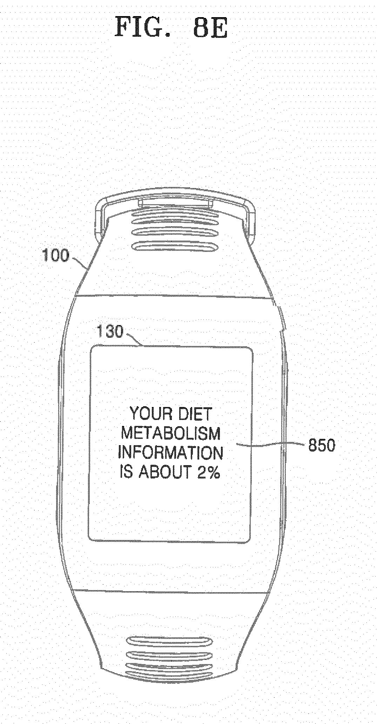

Also, the health care apparatus 100 may provide the diet metabolism information. For example, as shown in FIG. 8E, a screen 850 about the diet metabolism information may be provided. The diet metabolism information may be indicated in a number. However, the exemplary embodiments are not limited thereto. The diet metabolism information may be displayed in an image.

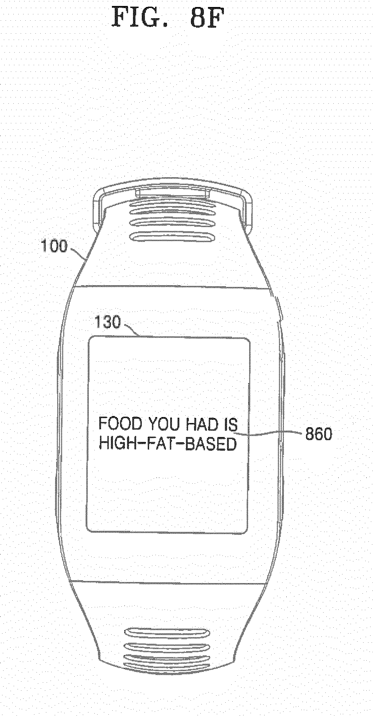

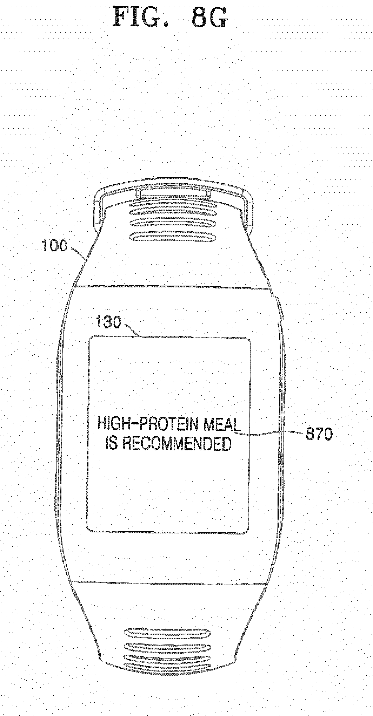

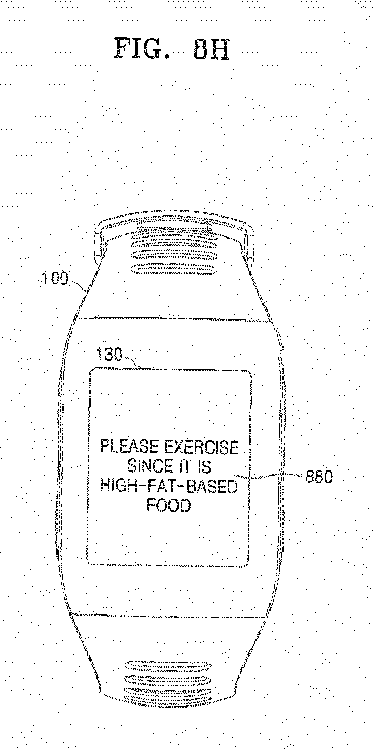

The health care apparatus 100 may provide information about a type of food or a guide about dietary habits or life habits, based on the diet metabolism information. For example, as shown in FIG. 8F, the health care apparatus 100 may provide a screen 860 about a type of food, for example, 'food you had is high-fat-based food'. Alternatively, as shown in FIG. 8G, the health care apparatus 100 may provide a screen 870 guiding dietary habits, such as 'high-protein meal is recommended'. Alternatively, the health care apparatus 100 may provide a screen 880 guiding life habits, such as 'please exercise since it is high-fat-based food', as shown in FIG. 8H.

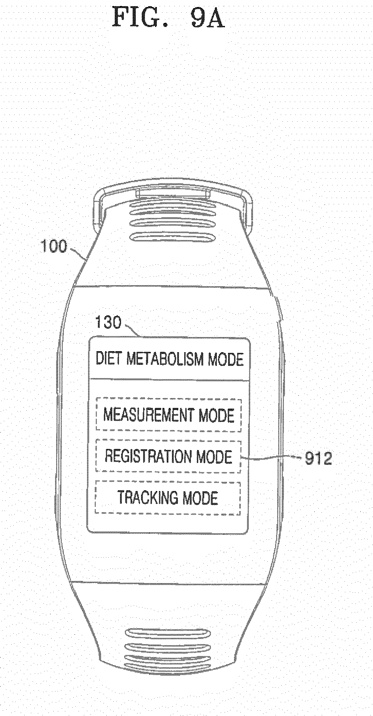

The health care apparatus 100 may track a health condition of the user by using the diet metabolism information. In order to track the health condition, the health care apparatus 100 may first register diet metabolism information corresponding to a reference or standard food, so as to associate the diet metabolism information with the standard food. FIGS. 9A through 9C are reference diagrams for describing a method of registering a standard food, according to an exemplary embodiment. For example, as shown in FIG. 9A, the health care apparatus 100 may provide diet metabolism modes. The user may input a command of selecting a registration mode 912 for registering a diet metabolism of a standard food. Then, the controller 120 of the health care apparatus 100 may activate the temperature sensor 110, and the temperature sensor 110 may detect a skin temperature of the user and transmit the skin temperature to the controller 120. Then, the controller 120 may generate a temperature waveform by using the skin temperature. The temperature sensor 110 may detect the skin temperature in real-time. Alternatively, the temperature sensor 110 may detect the skin temperature at regular intervals, for example, every 5 minutes, 10 minutes, 30 minutes, or 1 hour.

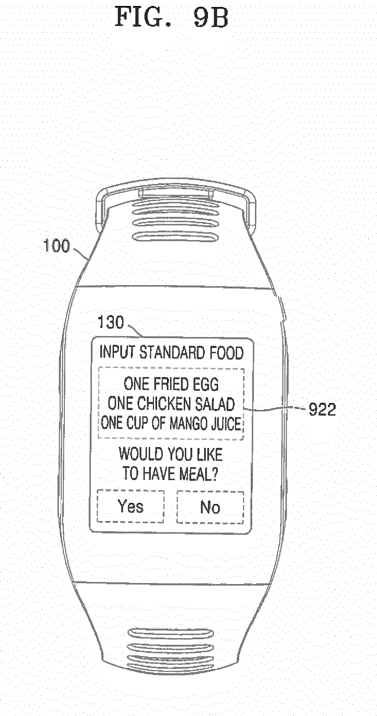

Meanwhile, the user may input information about the standard food through the user interface 150. For example, as shown in FIG. 9B, the user may input one fried egg, one chicken salad, and one cup of mango juice as information 922 about standard foods. Also, the user may input a user command indicating that a meal is starting. Here, the standard foods may be foods in which a plurality of nutrients are mixed, but the exemplary embodiments are not limited thereto. For example, the standard foods may contain a single nutrient.

Also, the health care apparatus 100 may generate a temperature waveform based on a point of time when a meal is started, and determine a reference temperature from the temperature waveform. Also, the health care apparatus 100 may determine a dietary temperature waveform from the reference temperature and the temperature waveform, and obtain diet metabolism information about the standard foods by using the dietary temperature waveform.

For example, the health care apparatus 100 may determine a dietary temperature and a dietary time from the dietary temperature waveform, obtain information about diet induced heat of the user by using the reference temperature, the dietary temperature, the dietary time, and physical information of the user, and obtain the diet metabolism information by using the information about the diet induced heat and information about the standard foods. The controller 120 may determine the diet metabolism information about the standard foods based on a ratio of the information about the diet induced heat with respect to the information about the standard foods.

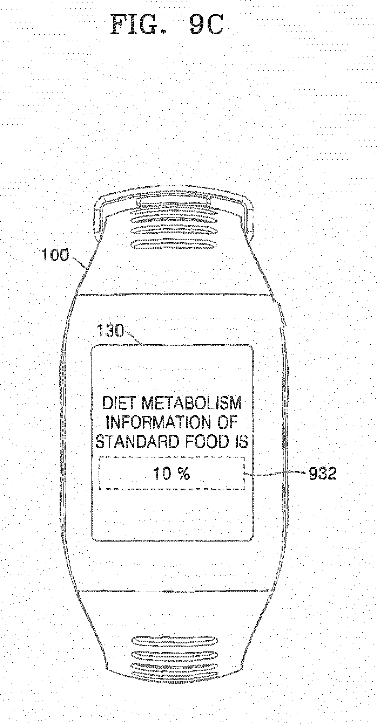

Also, as shown in FIG. 9C, the health care apparatus 100 may provide diet metabolism information 932 about the standard foods. The health care apparatus 100 matches and stores the standard foods and the diet metabolism information to register the diet metabolism information regarding the standard foods.

The user may check diet metabolism information according to time by periodically consuming a standard food since even if the user consumes the standard food, the diet metabolism information may decrease when the user is in poor health. Accordingly, the health care apparatus 100 may track a health condition of the user by using the diet metabolism information according to time.

For example, a mode of the health care apparatus 100 may be set to a tracking mode for tracking the health condition, according to a user input. When the tracking mode is set, the health care apparatus 100 may activate the temperature sensor 110, and provide a screen for determining whether a meal is started, together with information about the standard food.

The user may prepare the standard food. Then, the user may input a user command of starting a meal, and then eat the standard food. Then, the health care apparatus 100 may generate a temperature waveform, and determine a reference temperature from the temperature waveform. Also, the health care apparatus 100 may determine a dietary temperature waveform from the temperature waveform, and obtain diet metabolism information of the standard food by using the dietary temperature waveform. The health care apparatus 100 may determine whether diet metabolism capability of the user is changed by comparing currently obtained diet metabolism information (or current diet metabolism information) and pre-registered diet metabolism information (or previous diet metabolism information). For example, the health care apparatus 100 may define a tracking result as a ratio of the current diet metabolism information with respect to the previous diet metabolism information.

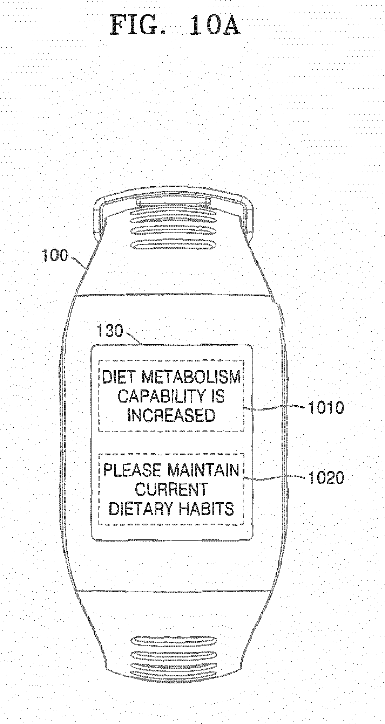

FIGS. 10A through 10C are reference diagrams for describing a method of displaying tracked diet metabolism information, according to an exemplary embodiment. For example, the health care apparatus 100 may define a tracking result as a ratio of current diet metabolism information with respect to previous diet metabolism information. When the tracking result is equal to or higher than 1, the health care apparatus 100 may determine that diet metabolism capability of a user is increased. Accordingly, as shown in FIG. 10A, the health care apparatus 100 may display an indicator 1010 indicating 'diet metabolism capability is increased', or display an indicator 1020 indicating 'please maintain current dietary habits'.

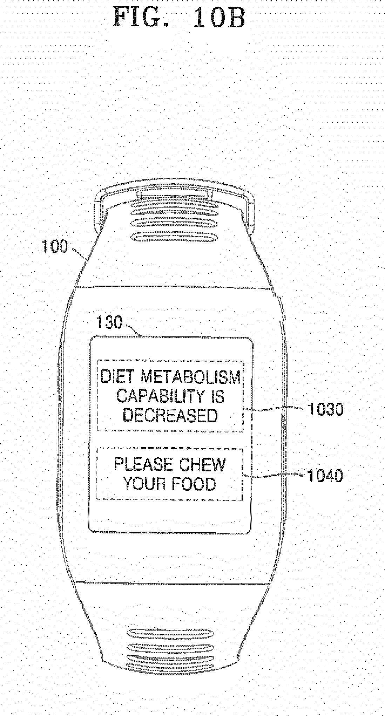

However, when the tracking result is lower than 1, the health care apparatus 100 may determine that the diet metabolism capability of the user is decreased. For example, as shown in FIG. 10B, the health care apparatus 100 may display an indicator 1030 indicating 'diet metabolism capability is decreased', or provide a dietary habit guide 1040 indicating 'please chew your food'.

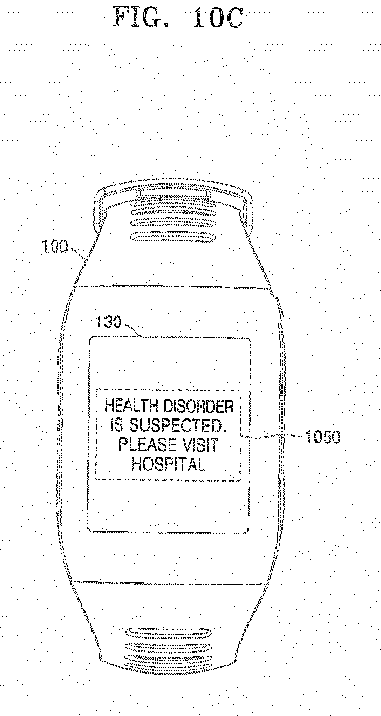

Meanwhile, the diet metabolism capability may decrease due to various diseases. Thus, when the tracking result is lower than or equal to a reference value, for example, 0.7, the health care apparatus 100 may determine that a health condition of the user is bad. Accordingly, when a degree of a diet metabolism change is lower than or equal to a reference value, the health care apparatus 100 may provide a phrase 1050 indicating 'health disorder is suspected. Please visit hospital', as shown in FIG. 10C.

Although not shown, when the user inputs a user command of selecting a user guide, the health care apparatus 100 may provide a GUI for making an appointment with a doctor by searching hospitals and examination departments related to a health condition, via the Internet. Alternatively, the health care apparatus 100 may automatically make an appointment with a doctor in a pre-set hospital.

Alternatively, when the tracking result is not related to a change of a health condition, the health care apparatus 100 may not display the tracking result, since the user may not be interested in all tracking results, but may be interested in factors that may adversely affect a health condition. The displaying of the tracking result may be determined by the user.

As described above, the health care apparatus 100 according to an exemplary embodiment may track the health condition of the user by using the diet metabolism information. For example, the standard food may contain a single nutrient. The health care apparatus 100 according to an exemplary embodiment may track metabolic capability of the user regarding the single nutrient. For example, when the standard food is glucose, the health care apparatus 100 according to an exemplary embodiment may predict metabolic capability of the user regarding glucose.

Hereinabove, the health care apparatus 100 uses food information to obtain diet metabolism information. However, the exemplary embodiments are not limited thereto. The health care apparatus 100 may determine a type of food consumed by a user by comparing a dietary temperature waveform corresponding to a standard food and a dietary temperature waveform corresponding to the food consumed by the user.

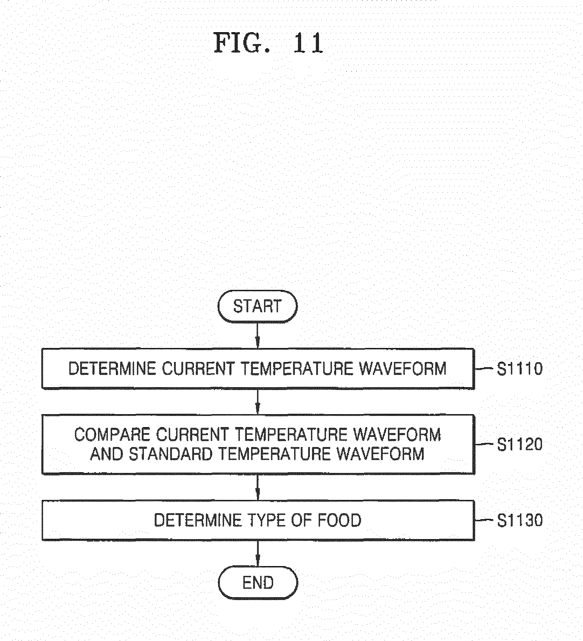

FIG. 11 is a flowchart of a method of determining a type of food by using a dietary temperature waveform, according to an exemplary embodiment. First, the health care apparatus 100 may pre-store a standard temperature waveform that is a dietary temperature waveform corresponding to a standard food, and the type of standard food. The standard food may contain a single nutrient, but the exemplary embodiments are not limited thereto. The standard food may contain a plurality of nutrients.

The health care apparatus 100 may register the standard food and the dietary temperature waveform corresponding to the standard food by using a method similar to the method of registering diet metabolism information of a standard food, which is described above. For example, when the user consumes protein, the health care apparatus 100 determines a first temperature waveform from a temperature waveform, when the user consumes carbohydrate, the health care apparatus 100 determines a second temperature waveform from the temperature waveform, and when the user consumes fat, the health care apparatus 100 determines a third temperature waveform from the temperature waveform.

The health care apparatus 100 may match and store a standard food and a dietary temperature waveform. As described above, when dietary temperature waveform is stored according to nutrients, the health care apparatus 100 may determine a type of food consumed by the user by using the dietary temperature waveform, without having to use physical information of the user.

The health care apparatus 100 may determine a dietary temperature waveform (or a current temperature waveform) corresponding to food currently consumed by the user, from a temperature waveform, in operation S1110. Since a method of determining the dietary temperature waveform has been described above, details thereof are not provided again.

Then, the health care apparatus 100 compares the current temperature waveform and a dietary temperature waveform (or a standard temperature waveform) corresponding to the standard food in operation S1120, and determines a type of the food consumed by the user by using a result of the comparing, in operation S1130. The health care apparatus 100 may determine the type of the food by using similarity between the current temperature waveform and the standard temperature waveform. The similarity may be cosine similarity. For example, a starting point of the standard temperature waveform and a starting point of the current temperature waveform may be matched in a coordinate space using time and a temperature as reference axes, and then the similarity may be determined based on a degree of similarity of vectors of corresponding dietary temperatures.

When similarities between the current temperature waveform and the first through third temperature waveforms are respectively 33%, 33%, and 33%, the health care apparatus 100 may determine that the types of foods consumed by the user have a ratio of protein, carbohydrate, and fat of 1:1:1. Alternatively, when the similarities between the current temperature waveform and the first through third temperature waveforms are respectively 50%, 25%, and 25%, the health care apparatus 100 may determine that the food consumed by the user is high-protein-based food.

Meanwhile, when the health care apparatus 100 is to obtain diet metabolism information, the health care apparatus 100 may receive food information through the user interface 150. However, the exemplary embodiments are not limited thereto. When the health care apparatus 100 includes a biosensor for detecting the food information, the user may not be required to input the food information.

The biosensor may detect the food information about the food consumed by the user. The biosensor may detect the food information via a noninvasive method. For example, the biosensor may detect the food information by using a Raman spectroscopy method, an infrared absorption method, or a radio frequency (RF) analyzing method. Since materials included in food have different molecular structures according to nutrients, a wavelength band of the materials may vary when light is irradiated on the materials. Accordingly, light may be irradiated on blood of the user, and the food information may be detected by analyzing a spectrum of light scattered or reflected by nutrients in the blood.

Alternatively, when the user consumes food, a status of the user is changed. For example, when the user consumes the food, viscosity in blood, flow of heat generated by digestion, particularity of constituents of blood, and transparency of blood are changed. The status of the user may be detected to detect the food information of the user. When the food information is predicted by detecting another component without directly detecting food components in blood, a database indicating relationships between foods and detected information may be used. The biosensor described above may be worn, for example, on a wrist, a breast, or an ankle of the user.

Also, a skin temperature of the user may change according to movement of the user. For example, when the user moves a lot, the skin temperature increases. Thus, the health care apparatus 100 may measure the skin temperature when the movement is within a certain range. When the movement is within the certain range, a change of the skin temperature according to the movement is low, and thus noise generated by the movement may be reduced.

Accordingly, the health care apparatus 100 according to an exemplary embodiment may further include a movement sensor detecting the movement of the user. The movement sensor may be an acceleration sensor, a gyro sensor, or a terrestrial magnetic sensor. Also, when a detecting result of the movement sensor is within a reference value, the controller 120 may activate the temperature sensor 110, generate a temperature waveform, and then determine a dietary temperature.

In addition, when the skin temperature of the user changes according to a heart rate, the health care apparatus 100 may further include a sensor for detecting the heart rate of the user, and may determine a temperature waveform when the heart rate is within a certain range as a waveform including a dietary temperature.

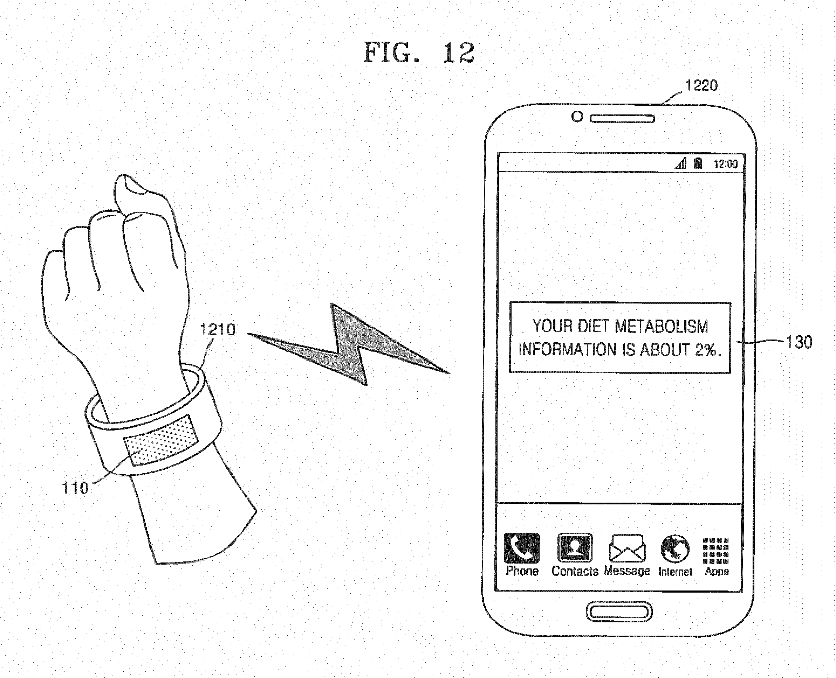

Also, the health care apparatus 100 may include a slave and a master. FIG. 12 is a diagram illustrating an example of a slave 1210 and a master 1220, according to an exemplary embodiment. The slave 1210 may include the temperature sensor 110 for detecting a skin temperature, and the master 1220 may include the display 130. Also, the slave 1210 and the master 1220 may each include a communication unit for mutual communication.

The slave 1210 may be a wearable device worn by a user. For example, the slave 1210 may be a wrist-type device that may be worn on a wrist of the user. The master 1220 may be a mobile phone, a smart phone, a desktop computer, a laptop computer, a tablet personal computer (PC), an electronic book terminal, a digital broadcasting terminal, a personal digital assistant (PDA), an internet protocol television (IPTV), a digital TV (DTV), or a health management server, but is not limited thereto.

Processing methods of the controller 120 that processes a personalized skin temperature may be written as computer programs and may be implemented in general-use digital computers including one or more processors that execute the programs using a computer-readable recording medium. Examples of the computer-readable recording medium include magnetic storage media (e.g., ROM, floppy disks, hard disks, etc.), optical recording media (e.g., CD-ROMs, or DVDs), etc.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments. While one or more exemplary embodiments have been described with reference to the figures, it will be understood by those of ordinary skill in the art that various changes in form and details may be made therein without departing from the scope as defined by the following claims.

**Claims**

1. A method of operating a health care apparatus, the method comprising:

    generating (S320) a temperature waveform according to time based on a skin temperature of the user;
    determining (S330), from the generated temperature waveform, a dietary temperature waveform corresponding to heat generated by food consumed by the user;
    determining (S610), from the dietary temperature waveform, a unit caloric value of the user by using a reference temperature, a dietary temperature and a dietary time, the unit caloric value is an amount of the heat generated by the food per unit surface area of the user, the reference temperature is a skin temperature of the user before heat is generated by the food, the dietary time is a time when heat is generated by the food;
    determining (S340) current diet metabolism information of the user by calculating a diet induced heat by multiplying the unit caloric value of the user for the dietary time and a body surface area of the user, the current diet metabolism information of the user is defined as a ratio of the diet induced heat with respect to a calorie intake;
    determining (S1130) a type of the food consumed by the user by using the current diet metabolism information
    outputting the current diet metabolism information (850); and
    displaying information (1010, 1030, 1050) about the type of food (860) consumed by the user in text or a graph.

2. The method of claim 1, wherein the reference temperature is a skin temperature of the user after the user has not consumed food for a predetermined amount of time; or an average skin temperature of the user during a predetermined period of time, in particular during a period of 30 minutes or less, based on a point of time when the user consumed the food.

3. The method of one of claims 1 to 2, wherein the determining (S340) the current diet metabolism information comprises:

    determining (S510) information about diet induced heat of the user by using the dietary temperature waveform and the physical information of the user; and
    determining (S520) the current diet metabolism information of the user by using the information about the diet induced heat and food information about the food consumed by the user; and
    wherein the determining (S510) the information about the diet induced heat in particular comprises:

        determining, from the generated temperature waveform, at least one of a reference temperature, a dietary temperature, and a dietary time;
        determining (S610, S620) the unit caloric value of the user by using the at least one of the reference temperature, the dietary temperature, and the dietary time; and
        determining (S630) the information about the diet induced heat of the user by using the unit caloric value of the user and the physical information of the user.

4. The method of claim 3, wherein the current diet metabolism information comprises a ratio of the information about the diet induced heat and the food information.

5. The method of one of claims 1 to 4, further comprising:

    detecting movement of the user, and

detecting the skin temperature of the user in response to a degree of the detected movement of the user being lower than or equal to a reference value.

6. A health care apparatus (100), comprising:

a temperature sensor (110) configured to determine a skin temperature of the user;
a display (130); and
at least one processor (120) configured to:

generate a temperature waveform according to time based on the skin temperature; and
determine, from the generated temperature waveform, a dietary temperature waveform corresponding to heat generated by food consumed by the user;

wherein the at least one processor (120) is further configured to:

determine, from the dietary temperature waveform, a unit caloric value of the user by using a reference temperature, a dietary temperature and a dietary time, the unit caloric value is an amount of the heat generated by the food per unit surface area of the user, the reference temperature is a skin temperature of the user before heat is generated by the food, the dietary time is a time when heat is generated by the food; determine a current diet metabolism information of the user by calculating a diet induced heat by multiplying the unit caloric value of the user for the dietary time and a body surface area of the user, the current diet metabolism information of the user is defined as a ratio of the diet induced heat with respect to a calorie intake; determine a type of the food consumed by the user by using the current diet metabolism information; and output information (1010, 1030, 1050) about the type of food consumed by the user via the display (130) in text or graph.

7. The health care apparatus of claim 6,
wherein the temperature sensor (110) is at least one of a thermistor, an integrated circuit, IC, temperature sensor, a quartz temperature sensor, a surface wave temperature sensor, an optical fiber sensor, a liquid temperature sensor, a thermopile, and a pyroelectric temperature sensor; or
wherein the temperature sensor (110) is further configured to receive the skin temperature of the user from an external device; or
wherein the temperature sensor (110) is further configured to determine the skin temperature based on heat radiated from skin of the user.

8. The health care apparatus of claim 6 or 7, wherein the at least one processor (120) is further configured to determine, from the temperature waveform, a reference temperature, and determine the dietary temperature waveform from the temperature waveform and the reference temperature.

9. The health care apparatus of claim 8, wherein the reference temperature is any one of a skin temperature of the user after the user has not consumed food for a predetermined amount of time and an average skin temperature of the user during a predetermined period of time based on a point of time when the user consumed the food.

10. The health care apparatus of one of claims 6 to 9, wherein the at least one processor (120) is further configured to determine information about diet induced heat of the user by using the dietary temperature waveform and the physical information of the user, and to determine the current diet metabolism information of the user by using the information about the diet induced heat and food information about the food consumed by the user.

11. The health care apparatus of claim 10, wherein the at least one processor (120) is further configured to:

determine, from the generated temperature waveform, at least one of a reference temperature, a dietary temperature, and a dietary time;
determine the unit caloric value of the user by using the at least one of the reference temperature, the dietary temperature, and the dietary time; and
determine the information about the diet induced heat of the user by using the unit caloric value of the user and the physical information of the user.

12. The health care apparatus of claim 10 or 11, wherein the current diet metabolism information comprises a ratio of the information about the diet induced heat and the food information.

13. The health care apparatus of one of claims 6 to 12, wherein the at least one processor (120) is further configured to generate the temperature waveform in response to a degree of movement of the user being lower than or equal to a reference value; and/or to determine the skin temperature of the user based on a temperature of skin of at least one of a wrist and an ankle of the user.

**Patentansprüche**

1. Verfahren zum Betreiben einer Gesundheitsvorsorgevorrichtung, wobei das Verfahren umfasst:

   Erzeugen (S320) einer Temperaturwellenform entsprechend der Zeit auf der Basis einer Hauttemperatur des Benutzers;
   Bestimmen (S330) einer Ernährungstemperatur-Wellenform entsprechend der durch vom Benutzer aufgenommener Nahrung erzeugten Wärme aus der erzeugten Temperaturwellenform;
   Bestimmen (S610) eines Kalorieneinheitswerts des Benutzers aus der Ernährungstemperatur-Wellenform durch Verwenden einer Referenztemperatur, einer Ernährungstemperatur und einer Ernährungszeit, wobei der Kalorieneinheitswert eine Menge der durch die Nahrung pro Oberflächeneinheit des Benutzers erzeugten Wärme ist, die Referenztemperatur eine Hauttemperatur des Benutzers ist, bevor Wärme durch die Nahrung erzeugt wird, und die Ernährungszeit eine Zeit ist, wenn Wärme durch die Nahrung erzeugt wird;
   Bestimmen (S340) einer aktuellen Ernährungsstoffwechsel-Information des Benutzers durch Berechnen einer ernährungsinduzierten Wärme durch Multiplizieren des Kalorieneinheitswerts des Benutzers für die Ernährungszeit und eine Körperoberfläche des Benutzers,
   wobei die aktuelle Ernährungsstoffwechsel-Information des Benutzers als ein Verhältnis der ernährungsinduzierten Wärme in Bezug auf eine Kalorienaufnahme definiert ist;
   Bestimmen (S1130) eines Typs der vom Benutzer aufgenommenen Nahrung durch Verwenden der aktuellen Ernährungsstoffwechsel-Information zur Ausgabe der aktuellen Ernährungsstoffwechsel-Information (850); und
   Anzeigen der Information (1010, 1030, 1050) zum Typ der vom Benutzer aufgenommenen Nahrung (860) als Text oder Graphik.

2. Verfahren nach Anspruch 1, wobei die Referenztemperatur eine Hauttemperatur des Benutzers ist, nachdem der Benutzer für eine vorgegebene Zeitmenge keine Nahrung aufgenommen hat; oder eine durchschnittliche Hauttemperatur des Benutzers während eines vorgegebenen Zeitraums, insbesondere während eines Zeitraums von 30 Minuten oder weniger, auf der Basis eines Zeitpunkts, zu dem der Benutzer die Nahrung aufgenommen hat, ist.

3. Verfahren nach Anspruch 1 oder 2, wobei das Bestimmen (S340) der aktuellen Ernährungsstoffwechsel-Information umfasst:

   Bestimmen (S510) einer Information zur ernährungsinduzierten Wärme des Benutzers durch Verwenden der Ernährungstemperatur-Wellenform und der physischen Information des Benutzers; und
   Bestimmen (S520) der aktuellen Ernährungsstoffwechsel-Information des Benutzers durch Verwenden der Information zur ernährungsinduzierten Wärme und der Nahrungsinformation zur vom Benutzer aufgenommenen Nahrung; und
   wobei das Bestimmen (S510) der Information zur ernährungsinduzierten Wärme insbesondere umfasst:

   Bestimmen von wenigstens einem Element der Gruppe umfassend eine Referenztemperatur, eine Ernährungstemperatur und eine Ernährungszeit aus der erzeugten Temperaturwellenform;
   Bestimmen (S610, S620) des Kalorieneinheitswerts des Benutzers durch Verwenden des wenigstens einen Elements der Gruppe umfassend die Referenztemperatur, die Ernährungstemperatur und die Ernährungszeit; und
   Bestimmen (S630) der Information zur ernährungsinduzierten Wärme des Benutzers durch Verwenden des Kalorieneinheitswerts des Benutzers und der physischen Information des Benutzers.

4. Verfahren nach Anspruch 3, wobei die aktuelle Ernährungsstoffwechsel-Information ein Verhältnis der Information zur ernährungsinduzierten Wärme und der Nahrungsinformation umfasst.

5. Verfahren nach einem der Ansprüche 1 bis 4, ferner umfassend: Erfassen der Bewegung des Benutzers, und

Erfassen der Hauttemperatur des Benutzers als Reaktion auf einen Grad der erfassten Bewegung des Benutzers, der kleiner gleich einem Referenzwert ist.

6. Gesundheitsvorsorgevorrichtung (100), umfassend:

einen zum Bestimmen einer Hauttemperatur des Benutzers konfigurierten Temperatursensor (110); eine Anzeige (130); und
wenigstens einen Prozessor (120) konfiguriert zum:

Erzeugen einer Temperaturwellenform entsprechend der Zeit auf der Basis der Hauttemperatur; und
Bestimmen einer Ernährungstemperatur-Wellenform entsprechend der durch vom Benutzer aufgenommener Nahrung erzeugten Wärme aus der erzeugten Temperaturwellenform;

wobei der wenigstens eine Prozessor (120) ferner konfiguriert ist zum:

Bestimmen eines Kalorieneinheitswerts des Benutzers aus der Ernährungstemperatur-Wellenform durch Verwenden einer Referenztemperatur, einer Ernährungstemperatur und einer Ernährungszeit, wobei der Kalorieneinheitswert eine Menge der durch die Nahrung pro Oberflächeneinheit des Benutzers erzeugten Wärme ist, die Referenztemperatur eine Hauttemperatur des Benutzers ist, bevor Wärme durch die Nahrung erzeugt wird, und die Ernährungszeit eine Zeit ist, wenn Wärme durch die Nahrung erzeugt wird;
Bestimmen einer aktuellen Ernährungsstoffwechsel-Information des Benutzers durch Berechnen einer ernährungsinduzierten Wärme durch Multiplizieren des Kalorieneinheitswerts des Benutzers für die Ernährungszeit und einer Körperoberfläche des Benutzers, wobei die aktuelle Ernährungsstoffwechsel-Information des Benutzers als ein Verhältnis der ernährungsinduzierten Wärme in Bezug auf eine Kalorienaufnahme definiert ist;
Bestimmen eines Typs der vom Benutzer aufgenommenen Nahrung durch Verwenden der aktuellen Ernährungsstoffwechsel-Information; und
Ausgeben einer Information (1010,1030,1050) zum Typ der vom Benutzer aufgenommenen Nahrung über die Anzeige (130) als Text oder Graphik.

7. Gesundheitsvorsorgevorrichtung nach Anspruch 6,
wobei der Temperatursensor (110) wenigstens ein Element der Gruppe umfassend einen Thermistor, eine integrierte Schaltung, einen Temperatursensor, einen Quartz-Temperatursensor, einen Oberflächenwellen-Temperatursensor, einen Lichtleitfasersensor, einen Flüssigkeitstemperatursensor, eine Thermosäule und einen pyroelektrischen Temperatursensor umfasst; oder
wobei der Temperatursensor (110) ferner zum Empfangen der Hauttemperatur des Benutzers von einer externen Vorrichtung konfiguriert ist; oder
wobei der Temperatursensor (110) ferner zum Bestimmen der Hauttemperatur auf der Basis der von der Haut des Benutzers abgestrahlten Wärme konfiguriert ist.

8. Gesundheitsvorsorgevorrichtung nach Anspruch 6 oder 7, wobei der wenigstens eine Prozessor (120) ferner zum Bestimmen einer Referenztemperatur aus der Temperaturwellenform und Bestimmen der Ernährungstemperatur-Wellenform aus der Temperaturwellenform und der Referenztemperatur konfiguriert ist.

9. Gesundheitsvorsorgevorrichtung nach Anspruch 8, wobei die Referenztemperatur eine beliebige von einer Hauttemperatur des Benutzers, nachdem der Benutzer für eine vorgegebene Zeitmenge keine Nahrung aufgenommen hat, und einer durchschnittlichen Hauttemperatur des Benutzers während eines vorgegebenen Zeitraums auf der Basis eines Zeitpunkts, zu dem der Benutzer die Nahrung aufgenommen hat, ist.

10. Gesundheitsvorsorgevorrichtung nach einem der Ansprüche 6 bis 9, wobei der wenigstens eine Prozessor (120) ferner zum Bestimmen einer Information zur ernährungsinduzierten Wärme des Benutzers durch Verwenden der Ernährungstemperatur-Wellenform und der physischen Information des Benutzers und zum Bestimmen der aktuellen Ernährungsstoffwechsel-Information des Benutzers durch Verwenden der Information zur ernährungsinduzierten Wärme und Nahrungsinformation zur vom Benutzer aufgenommenen Nahrung konfiguriert ist.

11. Gesundheitsvorsorgevorrichtung nach Anspruch 10, wobei der wenigstens eine Prozessor (120) ferner konfiguriert ist zum:

17

Bestimmen von wenigstens einem Element der Gruppe umfassend eine Referenztemperatur, eine Ernährungstemperatur und eine Ernährungszeit aus der erzeugten Temperaturwellenform;

Bestimmen des Kalorieneinheitswerts des Benutzers durch Verwenden des wenigstens einen Elements der Gruppe umfassend die Referenztemperatur, die Ernährungstemperatur und die Ernährungszeit; und

Bestimmen der Information zur ernährungsinduzierten Wärme des Benutzers durch Verwenden des Kalorieneinheitswerts des Benutzers und der physischen Information des Benutzers.

12. Gesundheitsvorsorgevorrichtung nach Anspruch 10 oder 11, wobei die aktuelle Ernährungsstoffwechsel-Information ein Verhältnis der Information zur ernährungsinduzierten Wärme und der Nahrungsinformation umfasst.

13. Gesundheitsvorsorgevorrichtung nach einem der Ansprüche 6 bis 12, wobei der wenigstens eine Prozessor (120) ferner konfiguriert ist zum Erzeugen der Temperaturwellenform als Reaktion auf einen Grad der Bewegung des Benutzers, der kleiner gleich einem Referenzwert ist, konfiguriert ist; und/oder zum Bestimmen der Hauttemperatur des Benutzers auf der Basis einer Hauttemperatur von einem Handgelenk oder/und einem Knöchel des Benutzers.

**Revendications**

1. Procédé de fonctionnement d'un appareil de soins de santé, le procédé comprenant les étapes consistant à :

générer (S320) une forme d'onde de température en fonction d'un temps sur la base d'une température cutanée de l'utilisateur ;

déterminer (S330), d'après la forme d'onde de température générée, une forme d'onde de température alimentaire correspondant à une chaleur générée par le produit alimentaire consommé par l'utilisateur ;

déterminer (S610), d'après la forme d'onde de température alimentaire, une valeur calorique unitaire de l'utilisateur en utilisant une température de référence, une température alimentaire et un temps alimentaire, la valeur calorique unitaire est une quantité de la chaleur générée par le produit alimentaire par unité de surface de l'utilisateur, la température de référence est une température cutanée de l'utilisateur avant que de la chaleur ne soit générée par le produit alimentaire, le temps alimentaire est un instant où la chaleur est générée par le produit alimentaire ;

déterminer (S340) des informations de métabolisme alimentaire actuel de l'utilisateur en calculant une chaleur induite par régime alimentaire en multipliant la valeur calorique unitaire de l'utilisateur pour le temps alimentaire et une surface corporelle de l'utilisateur, les informations de métabolisme alimentaire actuel de l'utilisateur sont définies comme un rapport de la chaleur induite par régime alimentaire par rapport à un apport calorique ;

déterminer (S1130) un type du produit alimentaire consommé par l'utilisateur en utilisant les informations de métabolisme alimentaire actuel et délivrer en sortie les informations de métabolisme alimentaire actuel (850) ; et afficher des informations (1010, 1030, 1050) sur le type de produit alimentaire (860) consommé par l'utilisateur sous forme de texte ou de graphique.

2. Procédé selon la revendication 1, dans lequel la température de référence est une température cutanée de l'utilisateur après que l'utilisateur n'ait pas consommé de produit alimentaire pendant une durée prédéterminée ; ou une température cutanée moyenne de l'utilisateur pendant une période de temps prédéterminée, en particulier pendant une période de 30 minutes ou moins, sur la base d'un instant où l'utilisateur a consommé le produit alimentaire.

3. Procédé selon l'une des revendications 1 à 2, dans lequel la détermination (S340) des informations de métabolisme alimentaire actuel comprend les étapes consistant à :

déterminer (S510) des informations sur la chaleur induite par régime alimentaire de l'utilisateur en utilisant la forme d'onde de température alimentaire et les informations physiques de l'utilisateur ; et

déterminer (S520) les informations de métabolisme alimentaire actuel de l'utilisateur en utilisant les informations sur la chaleur induite par régime alimentaire et les informations alimentaires sur le produit alimentaire consommé par l'utilisateur ; et

dans lequel la détermination (S510) des informations sur la chaleur induite par régime alimentaire comprend en particulier les étapes consistant à :

déterminer, d'après la forme d'onde de température générée, au moins l'un parmi une température de référence, une température alimentaire, et un temps alimentaire ;

déterminer (S610, S620) la valeur calorique unitaire de l'utilisateur en utilisant le au moins un parmi la température de référence, la température alimentaire, et le temps alimentaire ; et
déterminer (S630) les informations sur la chaleur induite par régime alimentaire de l'utilisateur en utilisant la valeur calorique unitaire de l'utilisateur et les informations physiques de l'utilisateur.

**4.** Procédé selon la revendication 3, dans lequel les informations de métabolisme alimentaire actuel comprennent un rapport entre les informations sur la chaleur induite par régime alimentaire et les informations de produit alimentaire.

**5.** Procédé selon l'une des revendications 1 à 4, comprenant en outre :

la détection d'un mouvement de l'utilisateur, et
la détection de la température cutanée de l'utilisateur en réponse à un degré du mouvement détecté de l'utilisateur qui est inférieur ou égal à une valeur de référence.

**6.** Appareil de soins de santé (100), comprenant :

un capteur de température (110) configuré pour déterminer une température cutanée de l'utilisateur ;
un affichage (130) ; et
au moins un processeur (120) configuré pour :

générer une forme d'onde de température en fonction d'un temps sur la base de la température cutanée ; et
déterminer, d'après la forme d'onde de température générée, une forme d'onde de température alimentaire correspondant à une chaleur générée par le produit alimentaire consommé par l'utilisateur ;
dans lequel le au moins un processeur (120) est en outre configuré pour :

déterminer, d'après la forme d'onde de température alimentaire, une valeur calorique unitaire de l'utilisateur en utilisant une température de référence, une température alimentaire et un temps alimentaire, la valeur calorique unitaire est une quantité de la chaleur générée par le produit alimentaire par surface unitaire de l'utilisateur, la température de référence est une température cutanée de l'utilisateur avant que de la chaleur ne soit générée par le produit alimentaire, le temps alimentaire est un instant où de la chaleur est générée par le produit alimentaire ;
déterminer des informations de métabolisme alimentaire actuel de l'utilisateur en calculant une chaleur induite par régime alimentaire en multipliant la valeur calorique unitaire de l'utilisateur pour le temps alimentaire et une surface corporelle de l'utilisateur, les informations de métabolisme alimentaire actuel de l'utilisateur sont définies comme un rapport de la chaleur induite par régime alimentaire par rapport à un apport calorique ;
déterminer un type du produit alimentaire consommé par l'utilisateur en utilisant les informations de métabolisme alimentaire actuel ; et
délivrer en sortie les informations des informations (1010, 1030, 1050) sur le type de produit alimentaire consommé par l'utilisateur via l'affichage (130) sous forme de texte ou de graphique.

**7.** Appareil de soins de santé selon la revendication 6,
dans lequel le capteur de température (110) est au moins l'un parmi une thermistance, un circuit intégré, IC, un capteur de température, un capteur de température à quartz, un capteur de température à ondes de surface, un capteur à fibre optique, un capteur de température de liquide, une thermopile, et un capteur de température pyroélectrique ; ou
dans lequel le capteur de température (110) est en outre configuré pour recevoir la température cutanée de l'utilisateur à partir d'un dispositif externe ; ou
dans lequel le capteur de température (110) est en outre configuré pour déterminer la température cutanée sur la base d'une chaleur rayonnée par la peau de l'utilisateur.

**8.** Appareil de soins de santé selon la revendication 6 ou 7, dans lequel le au moins un processeur (120) est en outre configuré pour déterminer, d'après la forme d'onde de température, une température de référence, et déterminer la forme d'onde de température alimentaire d'après la forme d'onde de température et la température de référence.

**9.** Appareil de soins de santé selon la revendication 8, dans lequel la température de référence est l'une quelconque parmi une température cutanée de l'utilisateur après que l'utilisateur n'ait pas consommé de produit alimentaire pendant une durée prédéterminée et une température cutanée moyenne de l'utilisateur pendant une période de temps prédéterminée sur la base d'un instant où l'utilisateur a consommé le produit alimentaire.

**10.** Appareil de soins de santé selon l'une des revendications 6 à 9, dans lequel le au moins un processeur (120) est en outre configuré pour déterminer des informations sur la chaleur induite par régime alimentaire de l'utilisateur en utilisant la forme d'onde de température alimentaire et les informations physiques de l'utilisateur, et pour déterminer les informations de métabolisme alimentaire actuel de l'utilisateur en utilisant les informations sur la chaleur induite par régime alimentaire et les informations de produit alimentaire sur le produit alimentaire consommé par l'utilisateur.

**11.** Appareil de soins de santé selon la revendication 10, dans lequel le au moins un processeur (120) est en outre configuré pour :

déterminer, d'après la forme d'onde de température générée, au moins l'un parmi une température de référence, une température alimentaire, et un temps alimentaire ;
déterminer la valeur calorique unitaire de l'utilisateur en utilisant le au moins un parmi la température de référence, la température alimentaire, et le temps alimentaire ; et
déterminer les informations sur la chaleur induite par régime alimentaire de l'utilisateur en utilisant la valeur calorique unitaire de l'utilisateur et les informations physiques de l'utilisateur.

**12.** Appareil de soins de santé selon la revendication 10 ou 11, dans lequel les informations de métabolisme alimentaire actuel comprennent un rapport entre les informations sur la chaleur induite par régime alimentaire et les informations de produit alimentaire.

**13.** Appareil de soins de santé selon l'une des revendications 6 à 12, dans lequel le au moins un processeur (120) est en outre configuré pour générer la forme d'onde de température en réponse à un degré de mouvement de l'utilisateur qui est inférieur ou égal à une valeur de référence ; et/ou pour déterminer la température cutanée de l'utilisateur sur la base d'une température de peau d'au moins l'un parmi un poignet et une cheville de l'utilisateur.

# FIG. 1

<u>100</u>

| | |
|---|---|
| 110 — TEMPERATURE SENSOR | 130 — DISPLAY |
| 120 — CONTROLLER | 140 — MEMORY |
| | 150 — USER INTERFACE |

# FIG. 2

FOOD INGESTION                    FOOD INGESTION

# FIG. 3

START

DETECT SKIN TEMPERATURE — S310

GENERATE TEMPERATURE WAVEFORM
FROM DETECTED SKIN TEMPERATURE — S320

DETERMINE DIETARY TEMPERATURE WAVEFORM
FROM TEMPERATURE WAVEFORM — S330

OBTAIN DIET METABOLISM INFORMATION BY
USING DIETARY TEMPERATURE WAVEFORM — S340

END

# FIG. 4A

FOOD INGESTION

FOOD INGESTION

410

$t_1$

$t_0$

T

# FIG. 4B

(i)

(ii)

# FIG. 5

START

OBTAIN INFORMATION ABOUT DIET INDUCED HEAT BY USING
DIETARY TEMPERATURE WAVEFORM AND PHYSICAL INFORMATION ──S510

IN DIET METABOLISM INFORMATION BY USING INFORMATION
ABOUT DIET INDUCED HEAT AND FOOD INFORMATION ──S520

END

# FIG. 6

START

OBTAIN UNIT CALORIC VALUE FROM
DIETARY TEMPERATURE WAVEFORM ──S610

OBTAIN UNIT CALORIC VALUE FOR DIETARY TIME ──S620

OBTAIN INFORMATION ABOUT DIET INDUCED HEAT BY
USING UNIT CALORIC VALUE AND PHYSICAL INFORMATION ──S630

END

FIG. 7

|  | DEFINITION | RATIO | TYPE OF FOOD |
|---|---|---|---|
| DIET METABOLISM INFORMATION (X) | DIET INDUCED HEAT / CALORIE INTAKE | 20% < X | PROTEIN MEAL |
|  |  | 10% < X < 20% | GENERAL MIXED MEAL |
|  |  | 3% < X < 10% | CARBOHYDRATE MEAL |
|  |  | X < 3% | FAT MEAL |

# FIG. 8A

# FIG. 8B

# FIG. 8C

# FIG. 8D

100

130

INPUT PHYSICAL
INFORMATION

840

HEIGHT : 175 cm

WEIGHT : 70 kg

# FIG. 8E

100

130

YOUR DIET
METABOLISM
INFORMATION
IS ABOUT 2%

850

# FIG. 8F

100

130

FOOD YOU HAD IS
HIGH-FAT-BASED — 860

# FIG. 8G

# FIG. 8H

# FIG. 9A

100

130

DIET METABOLISM MODE

MEASUREMENT MODE

REGISTRATION MODE — 912

TRACKING MODE

# FIG. 9B

100

130

INPUT STANDARD FOOD

ONE FRIED EGG
ONE CHICKEN SALAD
ONE CUP OF MANGO JUICE

922

WOULD YOU LIKE
TO HAVE MEAL?

Yes    No

# FIG. 9C

DIET METABOLISM
INFORMATION OF
STANDARD FOOD IS

10 %

# FIG. 10A

DIET METABOLISM
CAPABILITY IS
INCREASED

PLEASE MAINTAIN
CURRENT
DIETARY HABITS

# FIG. 10B

100

130

DIET METABOLISM
CAPABILITY IS
DECREASED — 1030

PLEASE CHEW
YOUR FOOD — 1040

# FIG. 10C

100

130

HEALTH DISORDER
IS SUSPECTED.
PLEASE VISIT
HOSPITAL

1050

# FIG. 11

START

DETERMINE CURRENT TEMPERATURE WAVEFORM — S1110

COMPARE CURRENT TEMPERATURE WAVEFORM
AND STANDARD TEMPERATURE WAVEFORM — S1120

DETERMINE TYPE OF FOOD — S1130

END

# FIG. 12

YOUR DIET METABOLISM
INFORMATION IS ABOUT 2%.

Phone  Contacts  Message  Internet  Appe

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140169400 A1